# EUROPEAN PATENT APPLICATION

(11) **EP 1 726 315 A1**
(43) Date of publication of application: **29.11.2006**
(21) Application number: 06425357.8
(22) Date of filing: 25.05.2006
(51) Int. Cl.: A61L 9/12, A01M 1/20

(54) **A device for the programmed and controlled dispensing of volatile substances for scenting the environment and/or chasing insects away**

(30) Priority: 26.05.2005 IT RM20050263
(71) Applicant: Faber S.p.A., 60044 Fabriano (Ancona) (IT)
(72) Inventor: Patarchi, Alberto, 00122 Ostia Lido (Roma) (IT)
(74) Representative: Lanzoni, Luciano

(57) **Abstract**

A device for the programmed and controlled release of volatile substances for scenting the environment and chasing insects away comprises a plurality of modular apparatuses set side by side, powered by a power supply (14) and controlled through a control unit (10), each apparatus comprising a small ventilator (6) for creating a flow of air (7) able to lap a support element (5) facing the ventilator (6) and supporting a disposable cartridge (9) able to emit a volatile substance.

## Description

The present invention relates to a device for the programmed and controlled dispensing of volatile substances for the neutralisation of bad odours, for scenting the environment and chasing insects away.

There are already several means for scenting environments or for neutralising the bad odours that are created therein or for chasing away annoying insects.

For example, the combustion of scented substances is used, such as incense sticks, or scented candles, or the heating of liquids or cartridges in electrical apparatuses that irradiate the scented essences contained therein, and other manners and means.

However, said manners and means for scenting the environment have the drawback of requiring a preparation on the user's part and not being programmable for their automatic operation during the day. Moreover, although the user can use means with different perfumes, it would be a large job to change them according to his/her desires.

The present invention aims to solve the aforementioned problems.

In particular, an object of the invention is to scent an environment in a desired manner, variable during the day.

Another object of the invention is to scent an environment in automatic, programmed fashion.

Yet another object is to allow in particular an automatic, programmed scenting in a home environment.

An additional object is together to enable the chasing away of annoying insects.

Yet an additional object is to allow a neutralisation of bad odours.

Therefore, according to the present invention a device is provided for the programmed and controlled dispensing of volatile substances for neutralising odours, scenting the environment and chasing insects away which, from a general point of view, is characterised in that it comprises a plurality of modular apparatuses set side by side, powered by a power supplied and controlled through a control unit, each apparatus comprising a small fan to create an air flow able to lap a support element, facing the fan and supporting a disposable cartridge able to emit a volatile substance.

The invention shall be described below in embodiments thereof, with reference to the accompanying drawings, in which:
Figure 1 is a schematic block diagram of a device according to the invention; and
Figure 2 is a schematic perspective view of a kitchen hood whereto the device according to the invention is applied.

With reference to the figures, it is shown in particular that the device according to the invention is incorporated in a kitchen hood 1. However, said placement of the device is not to be considered in any way to be limiting, both because said device can be extracted from the hood and positioned in other environments, and because it can be autonomous therefrom and placed wherever it is desired.

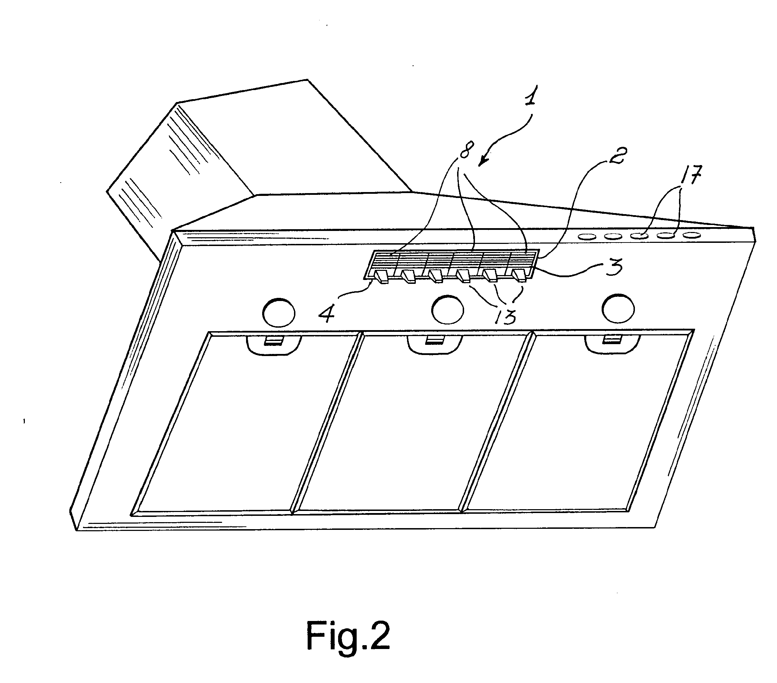

In the embodiment provided herein a device 3 for dispensing volatile substances according to the invention is provided at the bottom 2 of the kitchen hood 1. The device 3 is constituted by a plurality of modular apparatuses set side by side, identifiable in Figure 2 through the individual keys 13 of a keypad 4 and respective grid doors 8. The device 3 is positioned next to filtering means 20 and to lighting apparatuses 21, positioned in such a way as to border with the bottom of the hood 1.

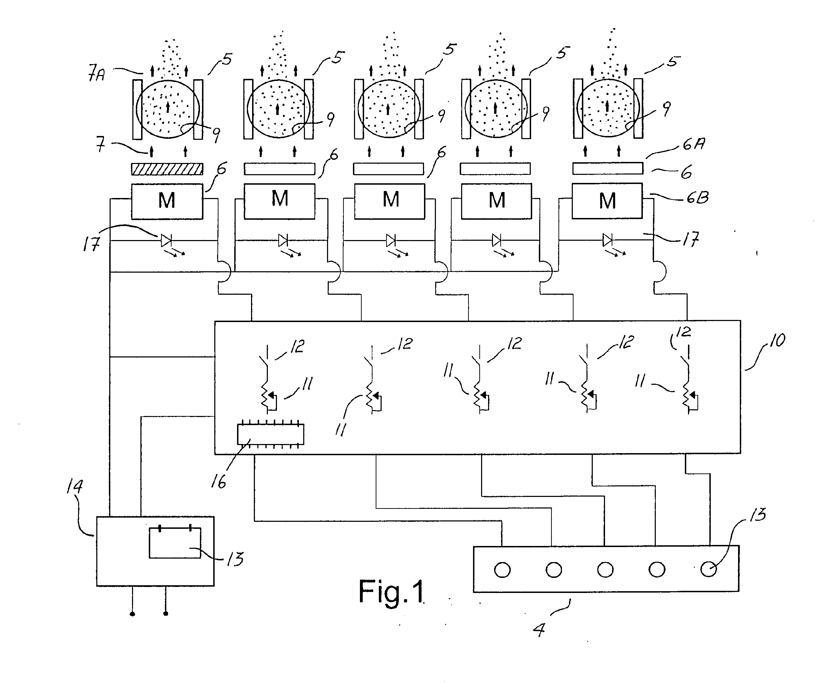

As shown in particular in Figure 1, which is a schematic representation of a block diagram of the device 1, said device 1 is powered by a power supply 14, provided for example with a buffer battery 15. The device is commanded from the keypad 4, whose keys 13 operate respective modular apparatuses for dispensing volatile substances (five in the described embodiment) substantially consisting of small ventilators 6. Each ventilator 6 comprises a small fan 6A and a micro-motor 6B.

The modular apparatuses are controlled by means of a control unit 10, comprising for each modular apparatus that composes the device, a power regulator 11 with relay contacts or semiconductors. The control unit further comprises a respective timer 12 for each modular apparatus. The control unit has a microprocessor 16, which can manage in appropriately programmed and automatic fashion the operation of the modular apparatuses.

Each fan 6A creates an air flow that laps a support element 5, facing the fan and able to support a cartridge 9. The cartridge 9 is formed by an aggregate of very fine powders, impregnated with a multiplicity of substances comprising scents, neutralising substances and insect repelling substances. The cartridge 9 is therefore capable of emitting a volatile substance, and it is discarded when it is depleted.

On each micro-motor is provided at least one operating indicator light 17.

Each fan 6A and related support 5 for the cartridge 9 are positioned facing the slotted door 8, openable to replace the cartridge 9 and able to allow the passage of air. The air flow is designated by the reference 7 before lapping the cartridge and 7A when, after lapping it, it carries away the volatile substances.

Positioning the device on the kitchen hood is particularly advantageous, although not exclusive, because the hood is the main element for air renewal in a home environment. However, as stated previously, the device according to the invention could be extracted from the hood and used as desired in other spaces, such as the bathroom or the living room, thanks to its buffer battery. Alternatively, the device according to the invention can be built in such a way as to constitute a unit that is independent of the hood.

In an exemplifying operation, the device according to the invention can, with one of its five modular apparatuses, neutralise bad odours, with another one reproduce the fragrance of freshly baked bread, with yet another one the aroma of coffee, with another modular apparatus mountain-air scents and with the last one provide the olfactory sensation of orange or lemon blossoms.

The emission can be selected using the keypad 4, or managed with a program controlled by the microprocessor 16 with regulation of the times, modes and quantities set by the user.

The automatic program of a typical day can be, with a greater number of modular apparatuses than the one mentioned above, and purely by way of example, the following: in the morning, aroma of chocolate, coffee and croissants; until noon, mountain air scent, as a relaxing fragrance; before lunch, aroma of freshly-baked bread, which stimulates the appetite; after cooking lunch, neutralisation of unpleasant odours; in the afternoon, scent of tea, meadow grass, roses and other relaxing scents; before dinner, aroma of rosemary *focaccia;* in the evening, scent of orange blossoms, oriental stimulating and inebriating scents. One of the keys with associated module can contain a mosquito repellent cartridge.

As stated, in addition to the range of fragrances usable through the program controlled by the microprocessor, the function of neutralising unpleasant odours is provided. For example, a timing can be selected that allows the emission of the neutralising substance at predetermined times (after lunch or dinner) and also provide the possibility of an automatic activation of said function when the hood is turned off.

The replacement of the cartridges, which can have a fairly long working life, is performed individually in the support elements 5.

The device according to the invention can be located in a hidden position under hood. The hood can be traditionally aspirating or filtering. With aspiration alone, residual odours are neutralised and preferred scents are propagated. With filtration, the hood also operates during cooking operations to eliminate unpleasant odours and right afterwards with the release of the scents. Among other matters, the modular apparatus with neutralisation cartridge can replace the traditional activated charcoal filter, disposal or washable, or ceramic able to be regenerated.

The above description refers to an embodiment of the invention and changes can be made within the scope of the appended claims.

## Claims

1. A device for the programmed and controlled release of volatile substances for neutralising odours, scenting the environment and chasing insects away, **characterised in that** it comprises a plurality of modular apparatuses set side by side, powered by a power supply (14) and controlled through a control unit (10), each apparatus comprising a small ventilator (6) for creating a flow of air (7) able to lap a support element (5) facing the ventilator (6) and supporting a disposable cartridge (9) able to emit a volatile substance.

2. Device as claimed in claim 1, **characterised in that** a control keypad (4) is provided for said control unit (10) comprising a series of selection keys (13), one for each respective modular apparatus, which, connected to the related ventilator (6), act with the air flow created thereby, on a cartridge (9).

3. Device as claimed in claim 1, **characterised in that** said control unit (10) is controlled by a microprocessor (16) in programmed, automatic fashion.

4. Device as claimed in claim 1, 3 and 4, **characterised in that** the control unit (10) comprises for each modular apparatus a regulator (11) of the power of the respective ventilator (6) and a timer (12) to determine its operation.

5. Device as claimed in any of the claims 1, 3 and 5, **characterised in that** each ventilator (6) has a fan (6A) actuated by a micro-motor (6B) whereon is provided at least one operation indicator light (17).

6. Device as claimed in any of the claims 1, 3, 5 and 6, **characterised in that** each ventilator (6) and related cartridge support (5) are positioned facing a slotted door (8), openable to replace the cartridge (9) and able to allow the passage of air.

7. Device as claimed in claim 7, cha 7, **characterised in that** it is mounted on a kitchen hood (1), comprising on its bottom (2) filtering means (20) and luminous apparatuses (21), the doors (8) of the modular apparatuses being also provided on said bottom.
